# EUROPÄISCHE PATENTSCHRIFT

(11) **EP 0 513 053 B1**
(45) Veröffentlichungstag und Bekanntmachung des Hinweises auf die Patenterteilung: **19.10.1994**
(21) Anmeldenummer: 91902673.2
(22) Anmeldetag: 24.01.1991
(51) Int. Cl.: C07C 65/11, C07C 51/12

(54) **VERFAHREN ZUR HERSTELLUNG VON 2-HYDROXY-NAPHTHALIN-6-CARBONSÄURE**
PROCESS AND DEVICE FOR PRODUCING 2-HYDROXY-NAPHTHALENE-6-CARBOXYLIC ACID
PROCEDE DE FABRICATION D'ACIDE CARBOXYLIQUE DE 2-HYDROXY-NAPHTALINE-6

(30) Priorität: 02.02.1990 DE 4003043
(43) Veröffentlichungstag der Anmeldung: 19.11.1992
(73) Patentinhaber: HOECHST AKTIENGESELLSCHAFT, 65926 Frankfurt am Main (DE)
(72) Erfinder: HAUTZEL, Volker, D-6093 Flörsheim am Main (DE); RITTNER, Siegbert, D-6082 Mörfelden-Walldorf (DE)
(86) Internationale Anmeldenummer: EP9100132
(87) Internationale Veröffentlichungsnummer: WO9111422

(56) Entgegenhaltungen:
- DE-C- 955 599
- US-A- 3 769 326
- US-A- 4 345 094

## Beschreibung

Die vorliegende Erfindung liegt auf dem technischen Gebiet von Zwischenprodukten, die beispielsweise zur Synthese von Azofarbstoffen und Polyestergrundstoffen eingesetzt werden können.

2-Hydroxy-naphthalin-6-carbonsäure stellt nicht nur einen wertvollen Synthesebaustein für Pharmazeutika, Textilhilfsmittel und Farbstoffe dar (s. bspw. Europäische Patentanmeldungs-Veröffentlichußg Nr. 0 292 955A), sondern insbesondere auch ein wichtiges Monomeres zur Herstellung von flüssigkristallinen Hochleistungskunststoffen und Fasern mit überragenden Eigenschaften (s. US-PS 4 393 191).

Technisch erfolgt die Synthese der 2-Hydroxy-naphthalin-6-carbonsäure analog der Verfahrensweise der sog. Kolbe-Schmitt-Reaktion, d.h. durch Umsetzung des Kaliumsalzes des β-Naphthols mit Kohlendioxid unter Druck bei 200-300°C (s. bspw. die US-Patentschriften Nrs. 1 593 816, 4 329 494 und 4 287 357). Die in diesen Veröffentlichungen beschriebenen Verfahrensweisen besitzen jedoch einige technische Nachteile; so entstehen beträchtliche Anteile an Zersetzungsprodukten, wie Teeren und Harzen, die sich ebenso wie die in Nebenreaktionen entstehenden 2-Hydroxy-naphthalin-3-carbonsäure und 2-Hydroxy-naphthalin-3,6-dicarbonsäure nur schwierig abtrennen lassen. Zwar wurde mit den in den Europäischen Patentanmeldungs-Veröffentlichungen Nrs. 0 053 824 und 0 081 753 beschriebenen Verfahrensweisen versucht, die Reaktionsführung zu verbessern und die Bildung der Zersetzungsprodukte zu vermindern, indem man in einem Lösemittel, bevorzugt Kerosin, arbeitet. Trotz dieses Vorteils gegenüber der lösungsmittelfreien Kolbe-Schmitt-Reaktion bleibt jedoch bei diesen beiden Verfahrensvarianten der grundsätzliche Nachteil, daß pro Mol entstandene 2-Hydroxy-naphthalin-6-carbonsäure jeweils 1 Mol β-Naphthol in der Reaktionsmasse unumgesetzt zurückbleibt, weswegen auch bei bestmöglicher Reaktionsführung die Ausbeute gemäß der Gleichung:

2 Mol Kalium-β-naphtholat + 1 Mol CO₂ → 1 Mol Dikalium-2-hydroxy-naphthalin-6-carbonsäure + 1 Mol β-Naphthol

auf maximal 50 % limitiert ist.

Bei der Herstellung der isomeren Verbindung 2-Hydroxy-naphthalin-3-carbonsäure hat man bereits vorgeschlagen, diesen Nachteil der unvollständigen Umsetzung des β-Naphthols zur Naphtholcarbonsäure dadurch zu vermeiden, daß man die Umsetzung von Kalium-β-naphtholat mit Kaliumcarbonat in Gegenwart von Kohlenmonoxid gemäß der Gleichung:

1 Mol Kalium-β-naphtholat + 1 Mol Kohlenmonoxid + 1 Mol K₂CO₃ → 1 Mol Dikalium-2-hydroxy-naphthalin-3-carbonsäure + 1 Mol Kaliumformiat

durchführt (s. Britische Patentschrift 1 155 776). Versucht man jedoch, diese Verfahrensweise mit dem Ziel der Herstellung von 2-Hydroxy-naphthalin-6-carbonsäure durch Variation der Reaktionsbedingungen Temperatur und Kohlenmonoxid-Druck zu modifizieren, so bleibt die Synthese dieses 6-Carboxy-Derivates ohne Erfolg.

Es wurde nun gefunden, daß man 2-Hydroxy-naphthalin-6-carbonsäure in überraschender Weise in guter Ausbeute und Reinheit erhält, wenn man die Umsetzung des Kalium-β-naphtholats mit Kaliumcarbonat und Kohlenmonoxid bei einer Temperatur von oberhalb 260°C, wie bei einer Temperatur zwischen 270°C und 360°C, bevorzugt zwischen 280 und 320°C, und bei einem Kohlenmonoxid-Druck von oberhalb 10 bar, wie bei einem CO-Druck von 50 bis 150 bar, bevorzugt zwischen 70 und 140 bar und insbesondere bei einem CO-Druck zwischen 80 und 120 bar, in Kaliumformiat als Lösemittel (Verdünnungsmittel) durchführt.

Die erfindungsgemäße Umsetzung in Kaliumformiat als Lösemittel bzw. Verdünnungsmittel (Kaliumformiat schmilzt oberhalb 167,5°C zu einer klaren Flüssigkeit) erfolgt nach der Reaktionsgleichung:

1 Mol Kalium-β-naphtholat + 1 Mol CO + 1 Mol K₂CO₃ → 1 Mol Dikalium-2-naphthol-6-carbonsäure + 1 Mol Kaliumformiat .

Das als Lösemittel (Verdünnungsmittel) dienende Formiat wird in mindestens der gleichmolaren Menge wie das Kalium-β-naphtholat-Ausgangsprodukt eingesetzt; die verwendete Menge an Kaliumformiat ist unkritisch und kann in weiten Grenzen variieren. Vorteilhaft ist es jedoch, Kaliumformiat in größeren Menge als Lösemittel (Verdünnungsmittel) einzusetzen, wie beispielsweise in der 2,5- bis 18-fachen, bevorzugt in der 6- bis 15-fachen, Gewichtsmenge des eingesetzten Kalium-β-naphtholats, um eine möglichst gute Durchmischung der im Kaliumformiat gelösten Reaktanten Kalium-β-naphtholat und Kaliumcarbonat und dem gasförmigen Kohlenmonoxid an den Phasengrenzen zu gewährleisten. Hierbei sind selbstverständlich, wie bei jeder in der Technik durchzuführenden Reaktion zwischen einer Gas- und einer Flüssigphase, die üblichen guten Durchmischungsmaßnahmen vorzunehmen, wie beispielsweise durch Einsatz hochintensiver Rührer, wie Turbinenrührer.

Das als Lösemittel (Verdünnungsmittel) eingesetzte Kaliumformiat bewirkt nicht nur eine hohe Selektivität der Reaktion unter Bildung des Kaliumsalzes der 2-Hydroxy-naphthalin-6-carbonsäure, sondern es hat auch den Vorteil, daß es im Reaktionsansatz kein Fremdprodukt darstellt, sondern identisch ist mit dem Nebenprodukt der Umsetzung. Das Kaliumformiat kann deshalb vollständig wieder in spätere Umsetzungen eingesetzt werden, oder aber man gewinnt aus dem Überschuß dieses Produktes durch Erhitzen mit konzentrierter Schwefelsäure Kohlenmonoxid, das in eine spätere Umsetzung zurückgeführt werden kann.

Das ebenso in die erfindungsgemäße Verfahrensweise eingesetzte Kaliumcarbonat wird in mindestens gleichmolarer Menge wie das Kalium-β-naphtholat in die Umsetzung eingesetzt. In der Regel liegt das molare Verhältnis von Kalium-β-naphtholat und Kaliumcarbonat zwischen 1:1 und 1:1,5.

Nach Beendigung der Synthese kann man den Ansatz auf verschiedene Weise aufarbeiten und die gebildete 2-Naphthol-6-carbonsäure isolieren. Eine Möglichkeit ist, das Reaktionsprodukt in Wasser zu lösen, mit konzentrierter Schwefelsäure auf einen pH-Wert von 7 einzustellen und das ausgefallene, nicht umgesetzte β-Naphthol abzufiltrieren. Das Filtrat wird mittels Schwefelsäure auf einen pH-Wert von 1 gestellt und die nunmehr ausgefallene rohe 2-Hydroxy-naphthalin-6-carbonsäure abgetrennt. Die Feinreinigung erfolgt analog bekannten Verfahrensweisen, beispielsweise durch Druckumlösung in Wasser oder durch Reinigung mit 1,4-Dioxan (s. hierzu bspw. Deutsche Offenlegungsschrift 38 00 989).

Die nachfolgenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

### Beispiel 1

10 Teile Kalium-β-naphtholat werden zusammen mit 7,6 Teilen Kaliumcarbonat und 75 Teilen Kaliumformiat in einem mit Rührwerk versehenen Edelstahl-Autoklaven gegeben, und das Ganze wird bei 230°C unter Rühren während 5 Stunden von Restfeuchte befreit. Sodann leitet man bei Raumtemperatur mit einem Druck von 50 bar Kohlenmonoxid ein und führt die Umsetzung bei einem Kohlenmonoxid-Druck von etwa 95 bar während etwa 5 Stunden unter intensivem Rühren bei 280°C durch.

Der Reaktionsansatz wird danach abgekühlt, der Autoklav entspannt, das Reaktionsprodukt in Wasser gelöst, mit konzentrierter Schwefelsäure auf einen pH-Wert von 7 gestellt, das ausgefallene, nicht umgesetzte β-Naphthol abfiltriert, das Filtrat auf einen pH-Wert von 1 mittels Schwefelsäure gestellt und die nunmehr ausgefallene 2-Hydroxy-naphthalin-6-carbonsäure isoliert. Die 2-Naphthol-6-carbonsäure kann aus dem Rohprodukt in üblicher Weise von den Nebenprodukten abgetrennt und somit rein erhalten werden.
- Ausbeute:: 34 % d.Th., bezogen auf das β-Naphthol als Ausgangsprodukt.
Die 2-Naphthol-3-carbonsäure wird zu 5 % als Nebenprodukt gebildet.

### Beispiel 2

Man verfährt gemäß der Verfahrensweise des Beispieles 1 mit dem Unterschied, daß man die Umsetzung bei 300°C und mit 125 Teilen Kaliumformiat durchführt. Man erhält die 2-Hydroxy-naphthalin-6-carbonsäure in einer Ausbeute von 40 % d.Th. neben 5,3 % 2-Naphthol-3-carbonsäure.

### Beispiel 3

Man verfährt gemäß der in den obigen Beispielen beschriebenen Verfahrensweise bei einem Kohlenmonoxid-Druck von 100 bar und einer Temperatur von 320°C während etwa 5 Stunden unter Einsatz von 10 Teilen Kalium-β-naphtholat, 7,6 Teilen Kaliumcarbonat und 75 Teilen Kaliumformiat. Nach Aufarbeitung des Reaktionsansatzes erhält man die 2-Hydroxy-naphthalin-6-carbonsäure in einer Ausbeute von 37,5 % d.Th. neben 5,7 % 2-Naphthol-3-carbonsäure.

### Beispiel 4

In einem mit einem Scheibenrührer versehenen Edelstahl-Autoklaven (ca. 5000 Volumenteile) gibt man 250 Teile Kalium-β-naphtholat, 190 Teile Kaliumcarbonat und 3500 Teile Kaliumformiat und erhitzt das Ganze unter Rühren während fünf Stunden bei 230°C zur vollständigen Entfernung von in den Ausgangsprodukten enthaltenem Wasser (Feuchte). Danach führt man unterhalb des Rührers Kohlenmonoxid bis zu einem Druck von 85 bar ein, erhöht die Temperatur des Ansatzes auf 300°C und führt die Umsetzung bei einem Kohlenmonoxid-Druck von etwa 100 bar noch etwa fünf Stunden bei einer Rührgeschwindigkeit von etwa 1100 U/min weiter.

Das Reaktionsprodukt wird sodann in der im Beispiel 1 angegebenen Verfahrensweise aufgearbeitet und gereinigt. Man erhält die 2-Hydroxy-naphthalin-6-carbonsäure in einer Ausbeute von 64 % d.Th..

## Patentansprüche

1. Verfahren zur Herstellung von 2-Hydroxy-naphthalin-6-carbonsäure bzw. dessen Dikaliumsalz, dadurch gekennzeichnet, daß man Kalium-β-naphtholat mit Kaliumcarbonat und Kohlenmonoxid bei einer Temperatur von oberhalb 260°C und bei einem Kohlenmonoxid-Druck von oberhalb 10 bar in Kaliumformiat als Lösemittel (Verdünnungsmittel) umsetzt und gegebenenfalls das gebildete Dikaliumsalz auf üblichem Wege in die 2-Hydroxy-naphthalin-6-carbonsäure überführt.

2. Verfahren nach Anspruch 1, dadurch gekennzeichnet, daß man die Umsetzung bei einer Temperatur zwischen 280 und 320°C durchführt.

3. Verfahren nach Anspruch 1 oder 2, dadurch gekennzeichnet, daß man die Umsetzung bei einem Kohlenmonoxid-Druck zwischen 70 und 140 bar durchführt.

## Claims

1. Process for the production of 2-hydroxy-naphthalene-6-carboxylic acid or its dipotassium salt, characterized by the fact that potassium-β-naphtholate is reacted with potassium carbonate and carbon monoxide at a temperature above 260°C and at a carbon monoxide pressure of above 10 bar in potassium formate as solvent (diluent) and, where appropriate, the dipotassium salt formed is converted in the usual manner to the 2-hydroxy-naphthalene-6-carboxylic acid.

2. Process in accordance with Claim 1, characterized by the fact that the reaction is carried out at a temperature between 280 and 320°C.

3. Process in accordance with Claim 1 or 2, characterized by the fact that the reaction is carried out at a carbon monoxide pressure between 70 and 140 bar.

## Revendications

1. Procédé pour préparer l'acide 2-hydroxy-naphtalène-6-carboxylique, ou ses sels dipotassiques, caractérisé en ce que l'on fait réagir le β-naphtolate de potassium avec le carbonate de potassium et l'oxyde de carbone à une température supérieure à 260°C et à une pression d'oxyde de carbone supérieure à 10 bars dans du formiate de potassium comme solvant (diluant) et en ce qu'éventuellement on transforme le sel dipotassique formé, de façon habituelle, en acide 2-hydroxy-naphtalène-6-carboxylique.

2. Procédé selon la revendication 1, caractérisé en ce que l'on effectue la réaction à une température entre 280 et 320° C.

3. Procédé selon la revendication 1 ou 2, caractérisé en que l'on effectue la réaction à une pression d'oxyde de carbone entre 70 et 140 bars.
